# EUROPEAN PATENT APPLICATION

(11) **EP 3 042 726 A1**
(43) Date of publication of application: **13.07.2016**
(21) Application number: 16158486.7
(22) Date of filing: 24.11.2010
(51) Int. Cl.: B08B 7/00, B08B 9/00, A01N 63/00, A61L 2/22

(54) **METHOD FOR THE MICROBIOLOGICAL CLEANING OF AN INTERIOR SPACE**

(30) Priority: 02.12.2009 BE 200900742
(62) Divisional of application: 10014957.4
(71) Applicant: METATECTA, naamloze vennootschap, 2845 Niel (BE)
(72) Inventor: Willocx, Filip Willem Maria, 3001 Heverlee (BE)
(74) Representative: Donné, Eddy

(57) **Abstract**

Method for the microbiological cleaning of patient and locker rooms, living spaces in hospitals, retirement and nursing homes, the method consisting of a first step, being the automatic atomization (3) of a biocide (2) that decontaminates and a following second step, being the atomization (3) of a benign biological culture (4) of benign aerobic spore formers for biological stabilization, whereby the atomization in both steps delivers microdrops of 5 to 10 microns.

## Description

The present invention relates to a method for the microbiological cleaning of patient and locker rooms, living spaces in hospitals, retirement and nursing homes.

More specifically, the invention is intended to clean an interior space that is biologically contaminated by viruses, bacteria, spores, yeasts or molds.

A disadvantage of these contaminating biological organisms is that bacteria can give rise to the formation of a biofilm, on interior space surfaces such as walls, ceilings or floors, but also on surfaces of objects such as kitchens or on furniture.

Biofilms are generated on surfaces that are moistened on a regular or less regular basis. Biofilm is composed of a structured community of microorganisms that are attached to an inert or living surface and that are enclosed by slime produced by themselves.

This sessial form is required to maintain a bacterial population on a specific spot (F. Haesebroeck et al. Vlaams Diergeneeskundig Tijdschrift, 2007, 76, pg 331-336).

Microorganisms that are located in such a biofilm, are on average much less sensitive to antimicrobial means, and can be up to 3000 X more resistant in the biofilm than in the free, planktonic form.
Known biofilmformers are *Pseudomonas* and *Serratia, Staphylococcus* and *Vibrio* species or yeasts, such as *Candida albicans.*

A disadvantage of traditional decontamination procedures is that they are often detrimental to the environment by the use of aggressive chemical antimicrobial means, that cause obnoxious odours and moreover are not efficient enough to fight microorganisms in the biofilm.

EP 1.283.010 defines a biofilm with resistant pathogenic microorganisms as a negative biofilm, and describes a composition of non pathogenic microorganisms that can be used to create a positive biofilm aimed at preventing hospital infections. The composition can be applied as an aqueous solution to surfaces in hospital rooms, for instance after each disinfection of a room or before a patient change in the room.

EP 0.852.114 describes a method to fight microorganisms pathogenic to animals by the use of benign biological cultures, applied as an aqueous solution or in the shape of a dry powder, to interior surfaces of buildings that shelter animals.

SU 1.235.896 describes a method of disinfection involving spraying the rooms of e.g. hospitals and operating theatres with a suspension of live organisms of a benign Bacillus strain to prevent the spreading of hospital infections.

Application was in aqueous solution.

It is known that peroxides can be applied as environmentally friendly biocides to decontaminate interior spaces.

In this way hydrogen peroxide combined with a very small concentration of peracetic acid is used in the commercial biocide Tevan-PANOX® 1816 of the company Tevan B.V. which is dispersed by means of an atomizer (Clim'oMedic apparatus of the company Metatecta N.V.) in an interior space in order to decontaminate it.

After the atomization time a standing time of 2 hours is in order in which all the atomized hydrogen peroxide particles, that measure about 5 to 10 microns form microdrops that slowly fall down.

In this way a microdrop of 3 microns takes more than 6 minutes to bridge a height difference of 2 meter. The hydrogen peroxide particles do not leave any residu, but the peracetic acid remains present.

A disadvantage of this decontamination method is that the treatment must be repeated over and over again, since the remaining non destroyed noxious organisms, such as resistant bacteria or spores, always again recolonise the surfaces.

Another disadvantage of this decontamination method is that the treated rooms cannot be entered as long as unreduced peroxide is still left behind in the interior space.

Another disadvantage of traditional decontamination methods is that odours of biological origin (urine odour, transpiration odour, mold odour) are not cleared by the decontamination.

The present invention aims at providing a solution for the prementioned and other disadvantages, by providing a method that consists of a first step, being the automatic atomization of a biocide that decontaminates, and thereafter a second step, being the atomization of a biological culture of benign aerobic spore formers that stabilize in a biological way, whereby the atomization in both steps delivers microdrops of 5 to 10 microns.

An advantage of the method according to the invention is that all contaminated areas are reached, such as cracks, corners or unattainable rims, such that the biocide by the automatic atomization penetrates everywhere in order to decontaminate, but also that the biocide is spread by the atomization in the air and the surrounding air is thereby decontaminated, after which the benign biological culture during the ensuing stabilization phase also penetrates everywhere to colonise the surfaces.

Another advantage of the method according to the invention is that the treatment does not have to be repeated over and over again, since the benign biological culture sees to it that the remaining, non destroyed organisms such as resistant bacteria or spores are displaced and do not recolonise the surfaces.

Preferably the second step is executed within one hour after the first step.

An advantage of the method is that it does not take long, and that the treated rooms can be used again after a few hours, since the possibly remaining peroxide after the first step is consumed by the benign biological culture of the second phase.

Preferably the biocide of the first step consists of one or more peroxides, such as a mixture of hydrogen peroxide and peracetic acid.

An advantage of the method is that peroxides are environmentally friendly biocides, that do not leave residues after reduction.

Preferably the benign sporeformers consist of Bacillus species, such as *Bacillus subtilis, Bacillus amyloliguefaciens, Bacillus pumilus, Bacillus licheniformis* and *Bacillus megaterium.*

An advantage of these benign spore formers is that they can displace remaining resistant bacteria and yeasts and molds and thereby prevent that the surfaces are recolonised by the noxious microorganisms.

Yet another advantage of the method according to the invention is that the quantities of biocide and of benign biological culture can be adjusted to one another as a function of the initial biological contamination.

Another advantage of the atomization method is that it is capable of washing the air itself, as was demonstrated by a test at the University of Louvain in which sterile water was atomized at a rate of 6 g/m³ and the reduction of moulds and yeasts in the air was measured after 15 minutes.

Initial values of 23 and 24 yeasts and moulds per 100 liter of air were reduced to 3, respectively 9 yeasts and moulds per 100 liters of air after 15 minutes (10 february 2010, Dr. Ir. Pieter Moons, University of Louvain)

Preferably a deodorizing agent is added to the benign biological culture during the second stabilization step. The deodorizing agent can be a complex forming agent and can be based on surfactants.

An advantage of this deodorization is that the combined approach not only fights the microorganisms, responsible for odours of biological origin, but also odours of non-biological origin.

If in the spaces odours are present of non-biological origin, such as for instance cigarette smoke, fire odour, soot, then a complex former can be added to the benign biological culture, based on surfactants, such as the commercial product Metatectyl AGM of Metatecta N.V. Metatactyl AGM is a neutral, non-allergenic, well scenting liquid based on surfactants. These surfactants contain a hydrophilic and a hydrophobic part. The hydrophobic parts attach themselves to oily and carbon-based molecules and form micelles with them, by orienting the hydrophilic parts towards the outside, thereby lowering the surface energy of the obnoxious molecules and thus neutralizing them.

This technique is called surfactant based complex forming and precipitation.

Metatectyl AGM in this way allows to fight the odours of tobacco, oilsmoke, combustion gases or smoke formed in wells of fire.

Preferably during the second stabilization step a compatible aromatic substance is still added to the benign biological culture, such as a commercial fragrance like Metascent of Metatecta N.V., such that a refreshing scent effect is obtained.

Optionally one can add the enzyme catalase during the second stabilization step, thereby even accelerating the decomposition of the peroxide.

If the enzyme catalase is used, the second stabilization step is preferably followed by a third atomization step, whereby only pure water is atomized to drops of 5 to 10 microns.

An advantage of this third atomization step with pure water, is that it cleans the air from any remaining protein traces from the catalase.

Interior spaces where the method according to the invention could be fruitfully applied are patient and locker rooms, living spaces in hospitals, retirement and nursing homes.

With the intention to better show the features of the invention, a preferred embodiment of the method according to the invention is described by way of example without any limitative character, with referral to the added figures, in which :
figure 1 schematically represents the first step of the method according to the invention;
figure 2 represents the second step of the method according to the invention;
figure 3 represents the interior space after cleaning.

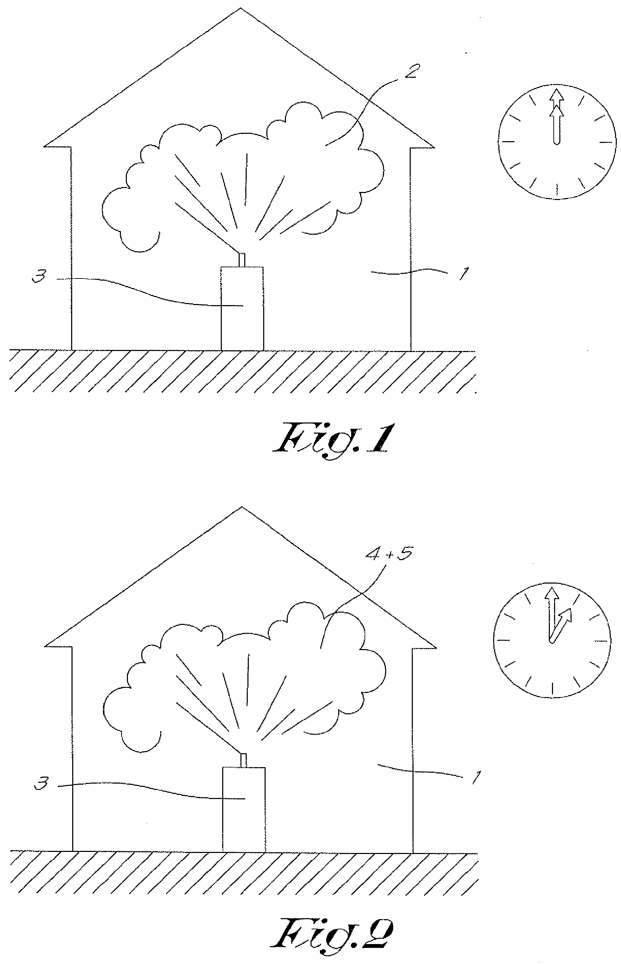

Figure 1 represents the first treatment of the interior space 1 with a peroxide 2 solution that is atomized by means of an atomizer 3.

Figure 2 represents the next treatment of the interior space 1 after maximally one hour, whereby a benign biological culture 4 of the type aerobic spore formers such as *Bacillus* species (e.g. *Bacillus subtilis, Bacillus amyloliguefaciens*, *Bacillus pumilus*, *Bacillus licheniformis, Bacillus megaterium* or any *Bacillus* spore former with a qualified presumption of safety (QPS) from the European Food Safety Authority (EFSA)) are also atomized in the same space.

Optionally a deodorizing agent 5 fitted to the origin or the odours is added to the benign biological culture.

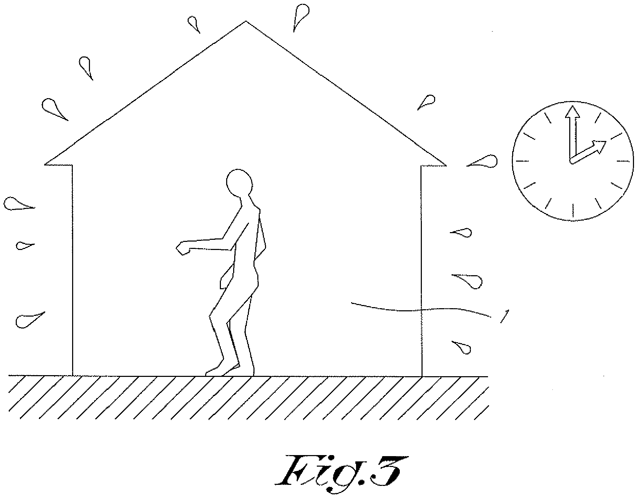

Figure 3 represents the cleaned and deodorized space after the treatments whereby the interior space is again accessible to inhabitants.

The application of this method can be described as follows.

In the first step noxious biological agents (viruses, hereditary material, bacteria, spores, yeasts and molds) are killed by the action of the biocide based on peroxide and this, in the space as well as on the biotic and non biotic surfaces.

The oxidizing peroxide is atomized by forming an aerosol with a specific drop size of 5 tot 10 micrometers that allows, on one hand, to obtain a homogeneous diffusion in the interior space and, on the other hand, to let the aerosol descend to disinfect the horizontal and vertical surfaces through microcondensation.

After maximally one hour the peroxide decontamination step I is followed by the biostabilization step II, whereby a benign biological solution of benign bacteria of the type aerobic spore former (such as *Bacillus subtilis* species) is also atomized in the same space, with the following action:
- The reduction of the remaining peroxide from step I to non damaging molecules (water and air) without residues.
- The homogeneous spreading of the bacteria through the standardized atomization in the space.
- The further destruction of the remaining microscopic biofilm, that was not completely destroyed by the biocide, by displacement with benign bacteria.
- The creation in situ of a stable and healty microflora that prevents recolonisation by other, pathogenic and/or decay inducing microorganisms after disinfection.

If odours are present in the space that have a biological origin (transpiration odour, mold odour, urine odour) the combination of the first and second step will already deal with the micro-organisms, that cause the odour change.

A special case of odours from a biological origin is presented by the decomposition of corpses such as in crime and trauma scene cleanup or in trash house cleanup.

In this case bacterial consumption of the corpse results in the formation of cadaverine (1,5 diaminopentane) and putrescine (1,4 diaminobutane) which are breakdown products of amino acids and proteins.

The first step with atomized peroxide will kill the bacteria, while step II involves the atomization of benign organisms to prevent new odour formation.

If odours are present in the space that are of non-biological origin (such as smoke, fire odour, soot or cigarettes) then a complex forming agent can be added to the benign biological solution in step II, based on surfactants.

These surfactants seek out oily substances such as tar and isolate them by means of micelle formation with hydrophilic groups at the outside. This micelle lowers the surface tension of these oily substances with neutralization of the odour as a consequence.

Besides this a compatible aromatic substance can also be added to the benign biological culture, such that a refreshing odour effect is obtained.

The present invention is in no way limited to the form of embodiment described by way of an example and represented in the figures, however, such a method can be realized in various forms without leaving the scope of the invention, as described in the following claims.

## Claims

1. Method for the microbiological cleaning of patient and locker rooms, living spaces in hospitals, retirement and nursing homes, whereby the method consists of a first step, being the automatic atomization of a biocide that decontaminates and a following second step, being the atomization of a benign biological culture of benign aerobic spore formers for biological stabilization, **characterized in that** the atomization in both steps delivers microdrops of 5 to 10 microns.

2. Method according to claim 1, **characterized in that** the second step is executed within one hour after the first step.

3. Method according to claim 1, **characterized in that** the biocide in the first step consists of one or a combination of peroxides.

4. Method according to claim 3, **characterized in that** the biocide consists of a mixture of hydrogen peroxide and peracetic acid.

5. Method according to claim 1, **characterized in that** the benign aerobic spore formers, consist of Bacillus species.

6. Method according to claim 5, **characterized in that** the Bacillus species consist of *Bacillus subtilis, Bacillus amyloliquefaciens, Bacillus pumilus, Bacillus licheniformis* or *Bacillus megaterium* or a combination of any of these species.

7. Method according to claim 5, **characterized in that** the Bacillus species is a Bacillus species with a qualified presumption of safety (QPS) from the European Food Safety Authority.

8. Method according to claim 1, **characterized in that** during the second step also a deodorizing agent is added to the benign biological culture.

9. Method according to claim 7, **characterized in that** the deodorizing agent consists of a complex forming agent.

10. Method according to claim 9, **characterized in that** the complex forming agent is based on surfactants.

11. Method according to claim 1, **characterized in that** during the second step also a compatible aromatic substance is added to the benign biological culture.

12. Method according to claim 11, **characterized in that** the aromatic substance consists of commercially available fragrances.
